**Europäisches Patentamt**

**European Patent Office**

**Office européen des brevets**

(19)

(11) Veröffentlichungsnummer: **0 205 176**
**B1**

(12) **EUROPÄISCHE PATENTSCHRIFT**

(45) Veröffentlichungstag der Patentschrift:
18.10.89

(51) Int. Cl.⁴: **A 61 B 17/00**

(21) Anmeldenummer: **86108019.0**

(22) Anmeldetag: **12.06.86**

(54) Chirurgisches Instrument.

(30) Priorität: **12.06.85 DE 3520960**

(43) Veröffentlichungstag der Anmeldung:
**17.12.86 Patentblatt 86/51**

(45) Bekanntmachung des Hinweises auf die Patenterteilung:
**18.10.89 Patentblatt 89/42**

(84) Benannte Vertragsstaaten:
**CH DE FR GB IT LI**

(56) Entgegenhaltungen:
**EP-A- 0 033 958**
**EP-A- 0 070 459**
**WO-A-84/01294**
**DE-A- 2 744 034**

(73) Patentinhaber: **AESCULAP AG, Möhringer Strasse 125, D-7200 Tuttlingen (DE)**

(72) Erfinder: **Wrobel, Walter-Gerhard, Dr. rer. nat., Stuttgarter Strasse 47, D-7200 Tuttlingen (DE)**

(74) Vertreter: **Hoeger, Stellrecht & Partner, Uhlandstrasse 14 c, D-7000 Stuttgart 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein chirurgisches Instrument zur Verwendung bei Operationen, bei denen die Strahlung eines $CO_2$-Lasers im Operationsgebiet auftrifft.

In zunehmendem Maße wird bei operativen Eingriffen Laserstrahlung eingesetzt, beispielsweise zum Durchtrennen von Gewebe oder zum Stillen von Blutungen. Es sind zu diesem Zweck Laser bekannt, die eine Strahlung im sichtbaren oder im nahen Infrarotbereich aussenden, zum Beispiel Nd-YAG-Laser mit einer Wellenlänge von 1,06 µm. Um bei diesen Strahlungen unbeabsichtigte Reflexionen der Strahlung an den bei der Operation verwendeten chirurgischen Instrumenten zu vermeiden, die aufgrund der engen Bündelung der Laserstrahlung zu Verletzungen des Patienten oder des Operateurs führen könnten, werden bei Operationen in dem genannten Wellenlängenbereich Instrumente verwendet, die eine schwarze Oberfläche haben, die also bei unbeabsichtigtem Auftreffen der Strahlung diese Strahlung weitgehend absorbieren.

Verwendet man jedoch Laser mit anderen Wellenlängen, beispielsweise einen $CO_2$-Laser mit einer Wellenlänge von 9 bis 12 µm, vorzugsweise 10,6 µm, stellt es sich heraus, daß derartige Instrumente mit schwarzer Oberfläche ungeeignet sind, da sie Strahlung mit der Wellenlänge eines $CO_2$-Lasers gerichtet reflektieren und da sie außerdem aufgrund der Strahlungsabsorption selbst heiß werden und daher Verletzungen hervorrufen können.

Aus EP-A-0 070 459 ist eine Haltevorrichtung für ein Lasermesser bekannt, bei der die Oberfläche dort aufgerauht ist, wo der Laserstrahl auftreffen kann.

Es ist Aufgabe der Erfindung, chirurgische Instrumente so auszubilden, daß sie bei Verwendung bei Operationen, bei denen die Strahlung eines $CO_2$-Lasers im Operationsgebiet auftrifft, weder unbeabsichtigte Reflexionen erzeugen noch durch eigene Aufheizung den Patienten verletzen, falls sie von der Strahlung eines $CO_2$-Lasers getroffen werden.

Diese Aufgabe wird beim chirurgischen Instrument der eingangs beschriebenen Art erfindungsgemäß dadurch gelöst, daß die Oberfläche des Instrumentes aus Gold, Nickel, Molybdän oder einer Kupfernickellegierung besteht und eine mittlere Rauhigkeit aufweist, die mindestens 10 µm beträgt.

Im Gegensatz zu dem bekannten Vorgehen bei Lasern im sichtbaren oder nahen Infrarotbereich wird also gemäß der Erfindung bewußt darauf geachtet, daß die Oberfläche des Instrumentes unbeabsichtigt auftreffende Strahlung möglichst vollständig reflektiert, so daß sich ein solches Instrument nicht durch Absorption der Laserstrahlung aufheizen kann. Hier wird also genau gegenteilig verfahren wie bei herkömmlichen Instrumenten, die bei den bekannten Laserstrahlungen im sichtbaren und nahen Infrarotbereich verwendet worden sind. Um aber gleichzeitig eine gerichtete Reflexion der Strahlung zu verhindern, wird die Oberfläche zusätzlich aufgerauht, wobei die mittlere Rauhigkeit mindestens in der Größenordnung der Wellenlänge der verwendeten Laserstrahlung liegt. Unter der mittleren Rauhigkeit versteht man dabei den Mittelwert der senkrechten Abweichung der Oberfläche von einer gedachten, vollkommen glatten Oberfläche, wobei diese Abweichung von der gedachten Oberfläche nach oben oder nach unten erfolgen kann. Wesentlich ist, daß diese Abweichungen möglichst statistisch verteilt sind, also kein regelmäßiges Muster ergeben. Bei einer solchen Oberfläche wird einfallende Strahlung nicht in eine bestimmte Richtung reflektiert, sondern in alle möglichen Richtungen gestreut, so daß bereits in kurzer Entfernung von dem Instrument die Leistungsdichte der reflektierten Strahlung so niedrig ist, daß keine Verletzungen mehr erfolgen können.

Diese Oberfläche aus Gold etc. weist die gewünschte hohe Reflexion auf, sie kann dabei auf eine entsprechend aufgerauhte Oberfläche aufgetragen sein, wobei auch die sehr dünne Schicht dann an ihrer Außenseite eine entsprechende Rauhigkeit aufweist, oder die Schicht kann selbst aufgerauht sein.

Grundsätzlich ist zwar das Streuverhalten einer aufgerauhten goldbeschichteten Oberfläche für $CO_2$-Laserstrahlung bekannt (J. Phys. E.: Sci. Instrum., Band 14, 1981, Seiten 1326 bis 1328), jedoch ist bisher nicht erkannt worden, daß gerade die Verwendung einer solchen Oberfläche für chirurgische Instrumente von besonderem Vorteil ist, die in einem Operationsbereich eingesetzt werden sollen, in dem $CO_2$-Laserlicht auftrifft.

Um auch bei sehr flachen Einfallwinkeln der Laserstrahlung eine möglichst vollständige Streuung zu erzielen, ist es vorteilhaft, wenn die mittlere Rauhigkeit der Instrumentenoberfläche mindestens 20 µm beträgt. Bei einer solchen Ausgestaltung kann erreicht werden, daß selbst bei einem Einfallwinkel von 80° gegenüber dem Einfallslot in einer bestimmten Richtung nur noch etwa 1 % der einfallenden Strahlung reflektiert wird.

Die Rauhigkeit der Instrumentenoberfläche kann auf verschiedene Weise erreicht werden, beispielsweise durch Sandstrahlen oder Kugelstrahlen des Instrumentes. Es ist auch möglich, die Oberfläche des Instrumentes durch Pulverbeschichtung oder durch Plasmaspritzen auf die gewünschte Rauhigkeit zu bringen.

Bei einem besonderen Ausführungsbeispiel ist die Oberfläche mit Diamantstaub beschichtet.

Es ist auch möglich, die Oberfläche durch elektrolytische Abscheidung der Oberflächenbeschichtung aufzurauhen.

Eine entsprechende Ausbildung der Oberfläche wird vorzugsweise für das gesamte chirurgische Instrument vorgesehen, um unerwünschte Aufheizeffekte und unerwünschte gerichtete Reflexionen vollständig zu vermeiden.

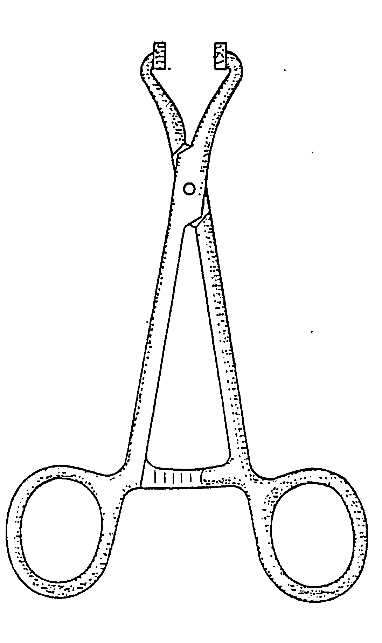

In der beigefügten Zeichnung ist ein chirurgisches Instrument dargestellt, dessen Oberfläche mit Gold beschichtet und derart aufgerauht ist, daß sich eine mittlere Rauhigkeit von mindestens 10 µm ergibt.

## Patentansprüche

1. Chirurgisches Instrument mit einer aufgerauhten Oberfläche zur Verwendung bei Operationen, bei denen die Strahlung eines $CO_2$-Lasers im Operationsgebiet auftrifft, dadurch gekennzeichnet, daß die Oberfläche des Instrumentes aus Gold, Nickel, Molybdän oder einer Kupfernickellegierung besteht und eine mittlere Rauhigkeit aufweist, die mindestens 10 µm beträgt.

2. Instrument nach Anspruch 1, dadurch gekennzeichnet, daß die mittlere Rauhigkeit mindestens 20 µm beträgt.

3. Instrument nach einem der voranstehenden Ansprüche, dadurch gekennzeichnet, daß die Oberfläche sand- oder kugelgestrahlt ist.

4. Instrument nach einem der Ansprüche 1 oder 2, dadurch gekennzeichnet, daß die Oberfläche pulverbeschichtet oder plasmagespritzt ist.

5. Instrument nach einem der Ansprüche 1 oder 2, dadurch gekennzeichnet, daß die Oberfläche durch elektrolytische Ablagerung aufgerauht ist.

## Claims

1. A surgical instrument with a roughened surface for use in operations in which the emission of a $CO_2$ laser strikes in the operation site, characterized in that the surface of the instrument is composed of gold, nickel, molybdenum or a copper nickel alloy and is of medium roughness which amounts to at least 10 µm.

2. An instrument according to Claim 1, characterized in that the medium roughness amounts to at least 20 µm.

3. An instrument according to one of the preceding Claims, characterized in that the surface is sandblasted or shot-blasted.

4. An instrument according to one of Claims 1 or 2, characterized in that the surface is powder coated or sprayed with plasma.

5. An instrument according to one of Claims 1 or 2, characterized in that the surface is roughened by electrolytic depositing.

## Revendications

1. Instrument chirurgical possédant une surface rendue rugueuse, destiné à être utilisé lors d'opérations où le rayonnement d'un laser $CO_2$ vient frapper le champ opératoire, caractérisé en ce que la surface de l'instrument est en or, nickel, molybdène ou un alliage de cuivre et de nickel et possède un rugosité moyenne d'au moins 10 µm.

2. Instrument selon la revendication 1, caractérisé en ce que la rugosité moyenne est d'au moins 20 µm.

3. Instrument selon une des revendications précédentes, caractérisé en ce que la surface est sablée ou grenaillée aux microbilles.

4. Instrument selon la revendication 1 ou 2, caractérisé en ce que la surface est revêtue par une poudre ou traitée par projection au plasma.

5. Instrument selon la revendication 1 ou 2, caractérisé en ce que la surface est rendue rugueuse par un dépôt électrolytique.